# EUROPEAN PATENT SPECIFICATION

(11) **EP 2 151 506 B1**
(45) Date of publication and mention of the grant of the patent: **17.04.2013**
(21) Application number: 09167522.3
(22) Date of filing: 10.08.2009
(51) Int. Cl.: C12Q 1/68

(54) **Method of amplifying target nucleic acid sequence by multiple displacement amplification including thermal cycling**
Verfahren zur Vervielfältigung einer Zielnukleinsäurensequenz durch mehrfache Strangverdrängungsamplifizierung unter thermischer Zyklisierung.
Procédé d'amplification d'une séquence d'acides nucléiques cible par une amplification de déplacement multiple comprenant un cycle thermique

(30) Priority: 08.08.2008 KR 20080078141
(43) Date of publication of application: 10.02.2010
(73) Proprietor: Samsung Electronics Co., Ltd., Suwon-si, Gyeonggi-do, 443-742 (KR)
(72) Inventor: Rhee, Joo-won, Gyeonggi-do (KR)
(74) Representative: Grünecker, Kinkeldey, Stockmair & Schwanhäusser

(56) References cited:
- YONG ZHANG D ET AL: "Amplification of target-specific, ligation-dependent circular probe" GENE, ELSEVIER, AMSTERDAM, NL, vol. 211, no. 2, 12 May 1998 (1998-05-12), pages 277-285, XP004120590 ISSN: 0378-1119
- VIGUERA E ET AL: "In vitro replication slippage by DNA polymerases from thermophilic organisms" JOURNAL OF MOLECULAR BIOLOGY, LONDON, GB, vol. 312, no. 2, 14 September 2001 (2001-09-14), pages 323-333, XP004449577 ISSN: 0022-2836
- WALKER G T: "EMPIRICAL ASPECTS OF STRAND DISPLACEMENT AMPLIFICATION" PCR METHODS & APPLICATIONS, COLD SPRING HARBOR LABORATORY PRESS, US, vol. 3, no. 1, 1 August 1993 (1993-08-01), pages 1-06, XP000600196 ISSN: 1054-9803
- SPARGO C A ET AL: "DETECTION OF M. TUBERCULOSIS DNA USING THERMOPHLIC STRAND DISPLACEMENT AMPLIFICATION" MOLECULAR AND CELLULAR PROBES, ACADEMIC PRESS, LONDON, GB, vol. 10, 1 January 1996 (1996-01-01), pages 247-256, XP002940177 ISSN: 0890-8508
- LITTLE M C ET AL: "ISOTHERMAL AMPLIFICATION OF DNA TARGETS BY STRAND DISPLACEMENT AMPLIFICATION" NONRADIOACTIVE LABELING AND DETECTION OF BIOMOLECULES, XX, XX, 1 January 1992 (1992-01-01), pages 218-223, XP009022640
- LOVMAR LOVISA ET AL: "Multiple displacement amplification to create a long-lasting source of DNA for genetic studies" HUMAN MUTATION, JOHN WILEY & SONS, INC, US, vol. 27, no. 7, 1 July 2006 (2006-07-01), pages 603-614, XP002508671 ISSN: 1059-7794
- KUMAR G ET AL: "Improved multiple displacement amplification with [phi]29 DNA polymerase for genotyping of single human cells" BIOTECHNIQUES, INFORMA LIFE SCIENCES PUBLISHING, WESTBOROUGH, MA, US, vol. 44, no. 7, 1 June 2008 (2008-06-01), pages 879-890, XP001538746 ISSN: 0736-6205

## Description

### BACKGROUND

### 1. Technical Field

Exemplary embodiments of the invention are directed to a method of amplifying a target nucleic acid sequence by multiple displacement amplification, and more particularly, to a method of amplifying a target nucleic acid sequence by multiple displacement amplification including thermal cycling.

### 2. Description of the Related Art

Various methods of amplifying a nucleic acid are known. Those methods include polymerase chain reaction (PCR), ligase chain reaction (LCR), self-sustained sequence replication (3SR), nucleic acid sequence-based amplification (NASBA), strand displacement amplification (SDA), amplification using Qβ replicase and multiple displacement amplification (MDA).

MDA is based on strand displacement amplification of a target nucleic acid sequence performed by a multiplex primer. In MDA, a replicated strand is generated and during replication, at least one replicated strand is displaced from the target nucleic acid sequence by strand displacement replication of another replicated strand. With regard to MDA, an available primer may include a primer set complementary to one strand of a target sequence and a primer set complementary to the other strand of the target sequence. Also, the primer may be a set of primers having a random sequence. Further, the primer may be a set of primers in which each member primer of the set is hybridized to only one strand of a target sequence.

A related art discloses a method of amplifying a target nucleic acid sequence, where the method includes bringing into contact a set of primers, DNA polymerase, and a target sample, and incubating the target sample under conditions that promote replication of the target sequence. The target sample is not subjected to denaturing conditions, and the replication of the target sequence results in replicated strands, in which during replication at least one of the replicated strands is displaced from the target sequence by strand displacement replication of another replicated strand.

MDA is performed at a substantially isothermal temperature and the incubation is performed at a sufficiently low temperature to promote hybridization of the primer with respect to the target sequence. That is, to promote hybridization of the primer, the incubation is performed at a temperature lower than the optimal temperature of a polymerase for its activity. As a result, amplification occurs only with a primer bound in the initial denaturing and annealing phases and at the corresponding location and thus, an amplification bias may occur, thereby reducing the amplification efficiency.

YONG ZHANG D ET AL: "Amplification of target-specific, ligation-dependent circular probe" GENE, ELSEVIER, AMSTERDAM, NL, vol. 211, no. 2, 12 May 1998 (1998-05-12), pages 277-285 discloses a polymerase chain reaction (PCR)-based gene amplification method using a circularizable oligodeoxyribonucleotide probe (C-probe). The C-probe contains two target complementary regions located at each terminus and an interposed generic PCR-primer binding region. The hybridization of the C-probe to a target brings the two termini in direct apposition. Subsequent ligation of the two termini results in a covalently linked C-probe that is of circular nature and allows for the generation of a multimeric single-stranded DNA via extension of the antisense primer by a strand displacement polymerase along the C-probe and displacement of a downstream strand, analogous to 'rolling circle' replication of bacteriophage, performed either isothermally or under cycling between a denaturation temperature, an extension temperature and an annealing temperature

VIGUERA E ET AL: "In vitro replication slippage by DNA polymerases from thermophilic organisms" JOURNAL OF MOLECULAR BIOLOGY, LONDON, GB, vol. 312, no. 2, 14 September 2001 (2001-09-14), pages 323-333 relates to *in vitro* replication slippage of DNA polymerases from thermophilic organisms. This publication discloses that different thermostable DNA polymerases undergo replication slippage *in vitro,* during single-round replication of a single-stranded DNA template carrying a hairpin structure. The slippage results in the generation of hairpin deletions during PCR amplifications. DNA polymerases that have a strand-displacement activity do not slip.

### SUMMARY OF THE INVENTION

Exemplary embodiments of the invention include a method of amplifying a target nucleic acid sequence by multiple displacement amplification (MDA) including thermal cycling.

Additional aspects will be set forth in part in the description which follows and, in part, will be apparent from the description, or may be learned by the practice of the presented embodiments.

Exemplary embodiments of the invention may include a method of amplifying a target nucleic acid sequence, wherein the method includes: bringing into contact a primer, a DNA polymerase, and a target nucleic acid sequence in a solution; and incubating the solution to replicate the target nucleic acid sequence, wherein the replication of the target nucleic acid sequence results in replicated strands and during replication, at least one of the replicated strands is displaced from the target sequence by strand displacement replication of another replicated strand, wherein the incubating is performed while thermal cycling is carried out at a temperature between an optimal temperature range of the DNA polymerase for its activity and a temperature range in which hybridization between the primer and the target nucleic acid sequence is promoted. "A primer" refers to a single primer as well as a set of 2 or more primers. "Thermal cycling" means to alternate the incubation temperature between at least two defined temperature ranges.

### BRIEF DESCRIPTION OF THE DRAWINGS

FIG. 1 is an electrophoretic image showing amplification results when a target sequence is amplified at 30°C or 60°C, or while thermal cycling is carried out between about 30°C and about 60°C.

FIG. 2 shows amplification results of the target sequence while thermal cycling is carried out between about 30°C and about 60°C in FIG. 1 and results obtained by using a control product.

### DETAILED DESCRIPTION OF EXEMPLARY EMBODIMENTS

Reference will now be made in detail to exemplary embodiments, examples of which are illustrated in the accompanying drawings, wherein like reference numerals refer to the like elements throughout. In this regard, the present embodiments may have different forms and should not be construed as being limited to the descriptions set forth herein. Accordingly, the embodiments are merely described below, by referring to the figures, to explain aspects of the present description.

A method of amplifying a target nucleic acid sequence according to an embodiment of the invention includes bringing into contact a primer or a set of primers, a DNA polymerase, and a target nucleic acid in a solution.

The primer may have a length of about 5 to about 20 bp. A melting temperature Tm may vary according to a length and nucleotide composition of the primer used and a component in a reaction solution, for example, a concentration of a cation. The term "melting temperature Tm" used herein refers to the temperature at which half of the strands of nucleic acid molecules among multiple copies of nucleic acid molecules are in the double stranded state and half are in the "random-coil" state. For example, when the primer has a length of about 5 to about 20 bp and a nucleotide of the primer is a natural nucleotide, Tm may be about 10°C to about 80°C. The primer may have a length of about 5 to about 8 bp. The primer may have a length of about 6 bp. The primer may include, in addition to the natural nucleotide, a modified nucleotide. For example, the primer may include at least one modified nucleotide and thus, is resistant to a nuclease, for example to an exonuclease. The modified nucleotide may be a biotinylated nucleotide, a fluorescent nucleotide, 5 methyl dCTP, 5-bromo-2'-deoxyuridine-5'-triphosphate (BrdUTP), or 5-(3-aminoallyl)-2'-deoxyuridine 5'-triphosphate. The primer may include a DNA or an RNA primer. Also, the primer may be labeled with a detectable label. The set of primers may include a plurality of primers. The set of primers may include 2 or more, for example, 3 or more primers, or 4 or more primers, or 5 or more primers, complementary to the same strand of the target nucleic acid. The set of primers may also include at least one primer complementary to the other stand of the target nucleic acid. The set of primers may include a plurality of primers and each primer may include a complementary portion, wherein the complementary portions of the primers are each complementary to a different portion of the target nucleic acid. The set of primers may include primers having a random nucleotide sequence. In an embodiment of the invention, the primer may be a random primer having a length of 5 bp, 6 bp, 7 bp, or 8 bp, or a mixture thereof.

An optimal temperature of the DNA polymerase for its activity may be higher than a hybridization temperature at which the primer is hybridized to the target sequence. In detail, the optimal temperature of the DNA polymerase may be higher than a denaturation temperature at which the primer hybridized to the target sequence is denatured In this case, a 'denaturation temperature' may be a temperature at which about 50% or more of the double strand is denatured, a temperature at which about 60% or more of the double strand is denatured, a temperature at which about 70% or more of the double strand is denatured, a temperature at which about 80% or more of the double strand is denatured, or a temperature at which about 90% or more of the double strand is denatured. That is, when a part of the double strand of the primer strand and the target sequence, for example, about 10%, about 20%, about 30%, about 40%, or about 50% or more is not denatured and remains, amplification may be improved through annealing of a new primer.

According to an embodiment of the invention, the term 'optimal temperature' may vary according to a condition, such as a reaction solution, but the optimal temperature of a polymerase used may be obvious to one of ordinary skill in the art under given buffer conditions. The term 'optimal temperature range' used herein may be the optimal temperature ±10°C, or the optimal temperature ±5°C, or the optimal temperature ±2.5°C. Preferably, the optimized temperature of the DNA polymerase is higher than a denaturation temperature at which the primer hybridized to the target sequence is denatured, and lower than a denaturation temperature at which a strand replicated by incubation within the temperature range in which hybridization between the primer and the target sequence is promoted, is denatured. The term 'denaturation temperature' is the same as described above.

According to an embodiment of the invention, the term 'hybridization temperature' refers to, unless additional description is provided, the temperature at which half of hybridizable sites are hybridized by hybridization between the primer and the target sequence. The hybridization temperature may be obvious to one of ordinary skill in the art through experiments or calculation. The hybridization temperature may be calculated by using a method disclosed in SantaLucia. & Hicks (2004) Annu. Rev. Biophys. Biomol. Struct. 33:415-40.

According to an embodiment of the invention, 'the temperature range in which hybridization between the primer and the target sequence is promoted' may be a temperature range in which hybridization between the primer and the target sequence is promoted. In this regard, such temperature range may be equal to or lower than the optimal temperature of the DNA polymerase-5°C, the optimal temperature of the DNA polymerase-10°C, or the optimal temperature of the DNA polymerase-15°C. "The temperature range in which hybridization between the primer and the target sequence is promoted" preferably is below the hybridization temperature at which half of the hybridizable sites are hybridized by hybridization between the primer and the target sequence".

According to an embodiment of the invention, the optimal temperature range of the DNA polymerase may be in the range of about 30°C to about 75°C, and the temperature range in which hybridization between the primer and the target sequence is promoted may be in the range of about 0°C to about 30°C. According to an embodiment of the invention, the optimal activation temperature range of the DNA polymerase may be in the range of about 40°C to about 65°C, and the temperature range in which hybridization between the primer and the target sequence is promoted may be in the range of about 0°C to about 30°C. The optimal temperature range of the DNA polymerase, for example, the optimal temperature range of a ϕ29 DNA polymerase may be about 32°C, the optimal temperature range of an exo(-) Bst DNA polymerase may be about 65°C, the optimal temperature range of a VENT^{™} exo- DNA polymerase may be about 75°C, the optimal temperature range of a 9°Nm DNA polymerase may be about 75°C, the optimal temperature range of a Klenow fragment may be about 37°C, and the optimal temperature range of a MMLV reverse transcriptase may be about 42°C.

The DNA polymerase is a polymerase that enables strand displacement replication, and may be a ϕ29 DNA polymerase, a Tts DNA polymerase, an M2 DNA polymerase, a VENT^{™} DNA polymerase, a T5 DNA polymerase, a PRD1 DNA polymerase, or a Bst DNA polymerase, but is not limited thereto.

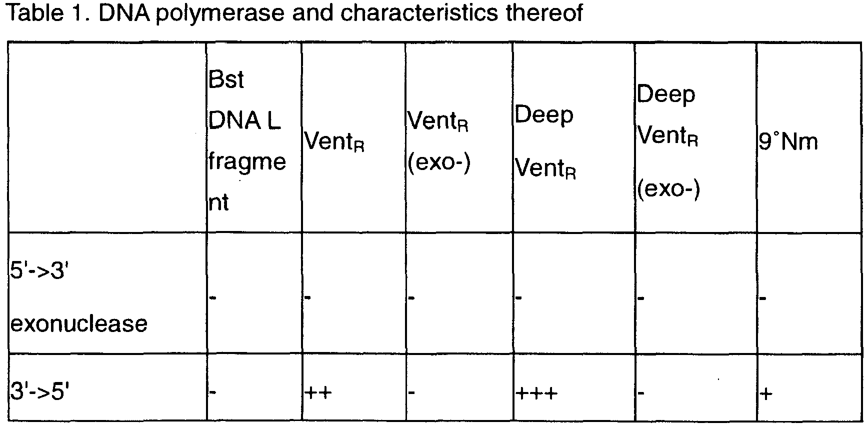

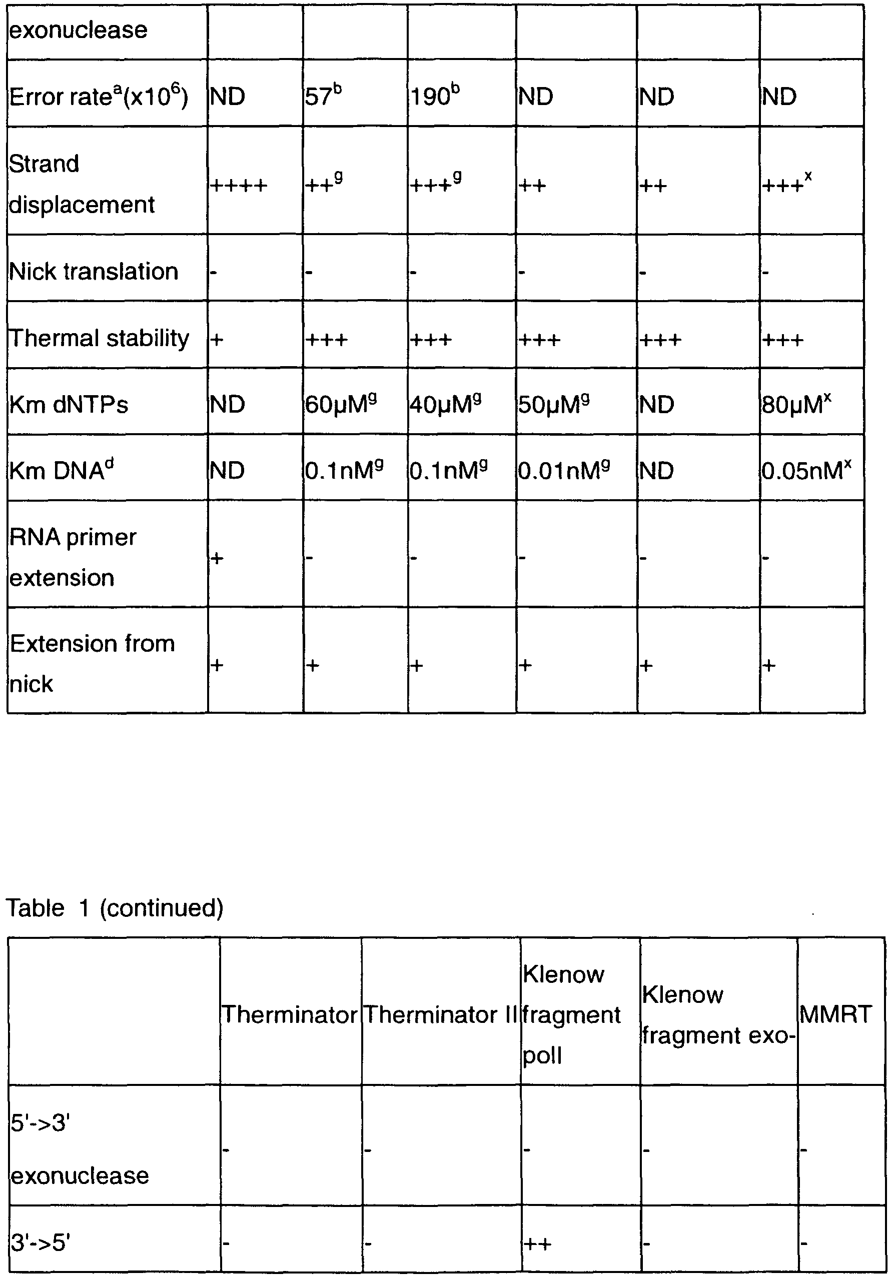

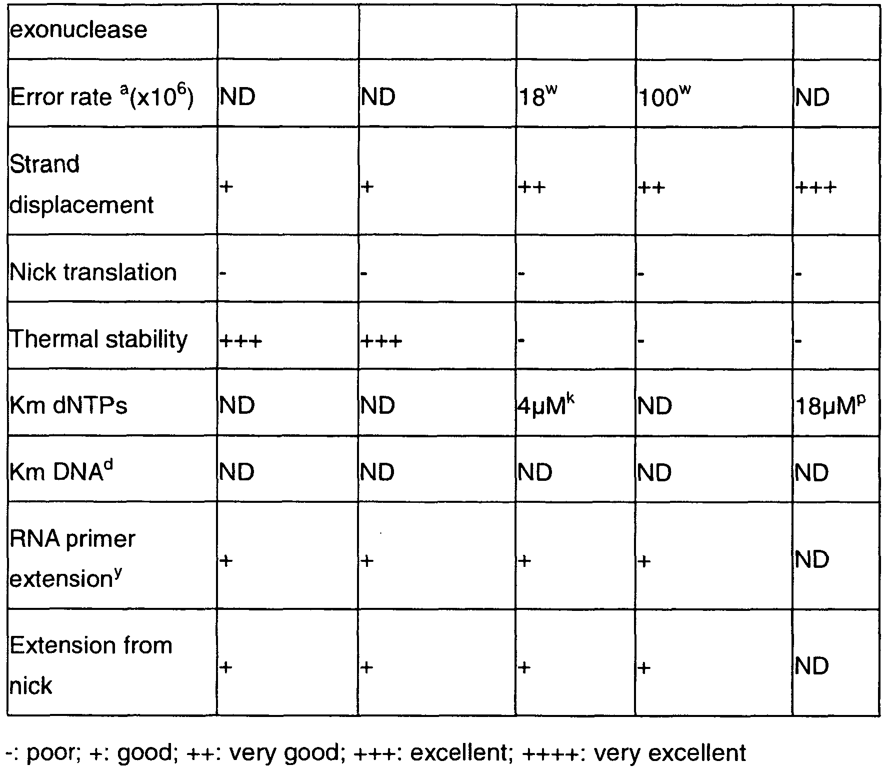

MMRT: M-MuIV reverse transcriptase, ND : not detected

Each of Km dNTP and Km DNA denotes a Michaelis constant with respect to dNTP and DNA.

a: Kunkel et al. (1987) Proc. Natl. Acad. Sci. USA, 84, 4865-4869

b: Mattila, P., Korpela, J., Tenkanen, T. and Pitkanen, K. (1991) Nucleic Acids Res., 19, 4967-4973

d: Km DNA is represented by a mole of a primer-template composite.

g: Kong, H.M., Kucera, R.B. and Jack, W.E., (1993) J. Biol. Chem., 268, 1965-1975.

k: Polesky, A.H., Steitz, T.A., Grindley, N.D.F. and Joyce, C.M. (1990) J. Biol. Chem., 265, 14579-14591.

p: Ricchetti, M. and Buc, H. (1990) EMBO J., 9, 1583-1593

x: Southworth, M.W. et al. (1996) Proc. Natl. Acad. Sci. USA, 93, 5281-5285.

w: Bebenek, K., Joyce, C.M., Fitzgerald, M.P. and Kunkel, T.A. (1990) J. Biol. Chem., 265, 13878-13887.

y: Amount of dNTP introduction into a RNA or DNA primer is compared to when a single-strand M13 DNA is used as a template.

According to an embodiment of the invention, the DNA polymerase may be the ϕ29 DNA polymerase, and the optimal temperature range may be about 30°C to about 34°C, for example about 32°C and the temperature at which hybridization is promoted may be about 4°C to about 22°C, for example about 20°C. In this case, the primer may be a random primer having a length of about 6 bp or a primer having a particular sequence. A "random primer" is a primer having an arbitrary sequence. A primer with a "particular sequence" is a primer that is complementary to and thus hybridizes to a defined region in the target sequence.

According to an embodiment of the invention, the DNA polymerase may be the exo(-) Bst DNA polymerase, the optimal temperature range may be about 46°C to about 75°C, for example about 65°C, and the temperature at which hybridization is promoted may be about 4°C to about 35°C, for example about 30°C. In this case, the primer may be a random primer having a length of about 6 bp or a primer having a particular sequence.

The target nucleic acid may be in the form of a material selected from the group consisting of blood, urine, semen, a lymphatic fluid, a cerebrospinal fluid, an amniotic fluid, a biopsy sample, a needle-aspiration biopsy, a maycer sample, a tumor sample, a tissue sample, a cell, cell debris, auxiliary debris, and combinations of at least two of the foregoing materials. The target nucleic acid may be a sample including the whole genome, or the whole genome itself.

The primer, the DNA polymerase, and the target nucleic acid may be brought into contact in an appropriate solution. The appropriate solution may vary according to the type of a polymerase used in amplification of a nucleic acid sequence, selected by one of ordinary skill in the art. For example, when the DNA polymerase is the ϕ29 DNA polymerase, the appropriate solution may be a solution including about 37mM Tris-HCI, pH 8.0, about 50 mM KCI, about 10 mM MgCl₂, and about 5 mM (NH₄)₂SO₄.

In a method according to an embodiment of the invention, bringing into contact may or may not include an initial operation that includes denaturing, for example, high-temperature denaturing the primer and the target sequence and annealing. However, except for the initial operation, the target sequence may not be exposed to conditions for denaturing the target sequence. If the polymerase is thermally stable, the denaturing and annealing may be performed in the presence of the polymerase.

A method according to an embodiment of the invention includes incubating the solution to replicate the target nucleic acid sequence. The incubating is performed while thermal cycling is carried out between an optimal temperature range of the DNA polymerase for its activity and a temperature range in which hybridization between the primer and the target sequence is promoted. The optimal temperature range of the DNA polymerase and the temperature range in which hybridization between the primer and the target sequence is promoted may be the same as described above. The thermal cycling may include incubating the solution in the 'optimal temperature range of the DNA polymerase' for a predetermined time period, for example, from about 30 seconds to about 6 hours, and incubating the solution in the 'temperature range in which hybridization between the primer and the target sequence is promoted' for a predetermined time period, for example, from about 30 seconds to about 3 minutes. In detail, the solution is first incubated in the 'temperature range in which hybridization between the primer and the target sequence is promoted' to induce annealing and elongation of the primer with respect to the target sequence, and then incubated in the 'optimal temperature range of the DNA polymerase.' However, the opposite case is also possible.

The incubating may be performed in a condition including a reaction component used for polymerization of a polymerase, such as dNTPs, ATP or salt.

In a method according to an embodiment of the invention, the incubating may be performed in a condition under which the target nucleic acid is not denatured.

In a method according to an embodiment of the invention, the replication of the target nucleic acid results in a replicated strand and during replication, at least one of the replicated strands is displaced from the target sequence by strand displacement replication of another replicated strand. As described above, a DNA polymerase used in an embodiment of the invention needs to be combined with a single or appropriate strand displacement factor that separates two annealed strands and displace a hybridized strand during replication. Hereinafter, the DNA polymerase will be referred to as a strand displacement DNA polymerase. The strand displacement DNA polymerase may not have 5'→3' exonuclease activity. The strand displacement is needed to synthesize a multiple copy of the target sequence. If the 5'→3' exonuclease activity exists, the synthesized strand may be destroyed. The strand displacement may be promoted by a strand displacement factor, such as helicase. The strand displacement DNA polymerase may be highly processive. Due to the strand displacement, replication is made from the multiple displacement copy, and such an amplification method is referred to as a multiple displacement amplification (MDA). Multiple displacement amplification is known in the art (see US 6,124,120).

According to an embodiment of the present invention, the target nucleic acid sequence includes DNA, RNA, and PNA. Also, the target nucleic acid sequence includes, in addition to natural nucleotides, a modified nucleotide. The target nucleic acid may be a double stranded or a single stranded nucleic acid.

A further embodiment of the present invention may include a method of amplifying a target nucleic acid sequence, the method comprising: bringing into contact a primer or a set of primers, a DNA polymerase, and a target nucleic acid sequence in a solution; and incubating the solution to replicate the target nucleic acid sequence, wherein the incubating is performed while thermal cycling is carried out between an optimal temperature range of the DNA polymerase for its activity and a temperature range in which hybridization between the primer and the target sequence is promoted, wherein the optimal temperature of the DNA polymerase is higher than a denaturation temperature at which the primer hybridized to the target sequence is denatured, and lower than a denaturation temperature at which a strand replicated by incubation within the temperature range in which hybridization between the primer and the target sequence is promoted, is denatured. Preferably, the replication of the target nucleic acid sequence results in a replicated strand and during replication, at least one of the replicated strands is displaced from the target sequence by strand displacement replication of another replicated strand.

A further embodiment of the present invention may include a method of amplifying a target nucleic acid sequence, the method comprising: bringing into contact a primer or a set of primers, a DNA polymerase, and a target nucleic acid sequence in a solution; and incubating the solution to replicate the target nucleic acid sequence, wherein the incubating is performed while thermal cycling is carried out between an optimal temperature range of the DNA polymerase for its activity and a temperature range in which hybridization between the primer and the target sequence is promoted, wherein the DNA polymerase comprises exo(-) Bst DNA polymerase, the optimal temperature range of the DNA polymerase is about 46°C to about 75°C, and the temperature range at which hybridization between the primer and the target sequence is promoted is in the range of about 4°C to about 35°C. Preferably, the replication of the target nucleic acid sequence results in a replicated strand and during replication, at least one of the replicated strands is displaced from the target sequence by strand displacement replication of another replicated strand.

The above embodiments will be described in further detail with reference to the following examples. These examples are for illustrative purposes only and are not intended to limit the scope of embodiments of the present invention.

Example 1: effect of thermal cycling on a multiple displacement amplification

In the current example, an exo(-) Bst DNA polymerase (New England Biolab) was used as a Bst DNA polymerase. The exo(-) Bst DNA polymerase, a hexamer random primer (Bioneer Co., Korea), and a human genome sample obtained by purifying blood using a blood DNA purification kit (Qiagen Co.) were brought into contact in a Bst DNA polymerase buffer (about 20 mM Tris-HCI, about 10 mM (NH₄)₂SO₄, about 10 mM KCI, about 2 mM MgSO₄, about 0.1% Triton X-100, pH about 8.8 at about 25°C) (New England Biolab), and then incubated at about 30°C or about 60°C, or while thermal cycling was carried out between about 30°C and about 60°C. When the thermal cycling was performed between about 30°C and about 60°C, the thermal cycling was repeatedly performed at 30°C for about 20 seconds and at about 60°C for about 40 seconds in a polymerase chain reaction (PCR) device until the time period indicated as above, that is, for about 0.5 hours, about 1 hour, about 1.5 hours, and about 2 hours was lapsed.

The exo(-) Bst DNA polymerase is known to have the optimal activity at a temperature of about 65°C (Mead, D.A. et al (1991) Biotechniques, 11, 76-87). After the reaction was finished, the obtained amplification product was electrophoresed in an about 1% gel by using an electrophoresis device, and then specifically labeled with SYBR Green I with respect to a double-strand DNA. Then, the resultant product was irradiated with an excitation light having a wavelength of about 480 nm and light emission was detected at 580 nm.

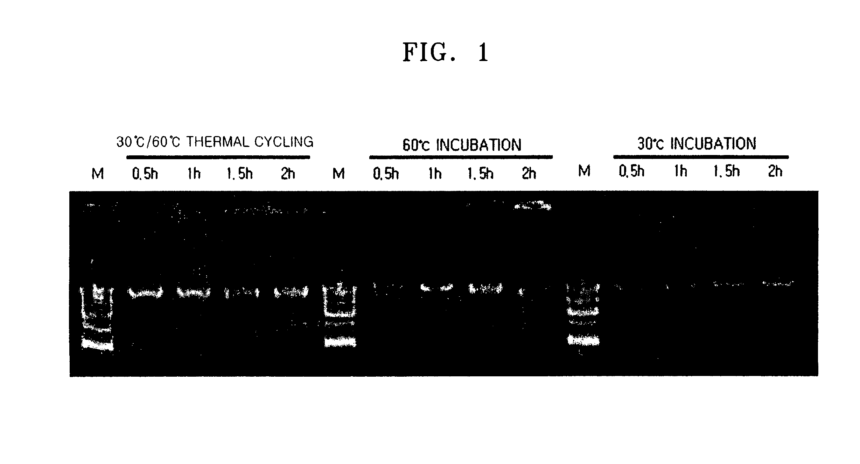

FIG. 1 is an electrophoretic image showing amplification results when a target sequence was amplified at about 30°C or about 60°C or while thermal cycling was carried out between about 30°C and about 60°C. Referring to FIG. 1, the target sequence was amplified more during a short time period when thermal cycling was carried out between about 30°C and about 60°C than when the reaction was carried out at 30°C or 60°C. In FIG. 1, M is a size marker, and 0.5 h, 1 h, 1.5 h, and 2 h respectively represent 0.5 hours, 1 hour, 1.5 hours, and 2 hours.

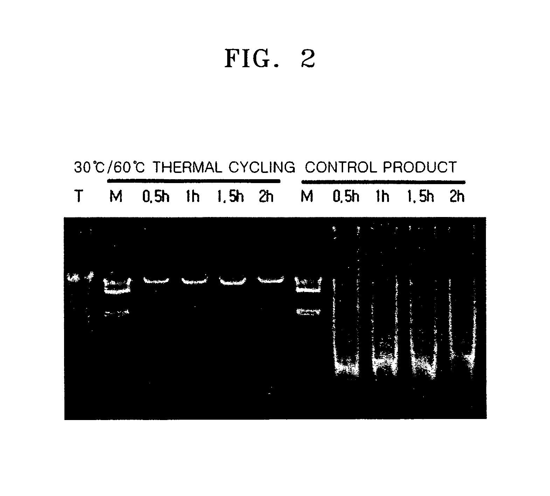

FIG. 2 shows amplification results of the target sequence while thermal cycling was carried out between about 30°C and about 60°C in FIG. 1 and results obtained by using a control product. The control product was REPLI-G ULTRA Kit (Qiagen Co.). Detailed reaction conditions will now be described in detail. A 50 ng template DNA was treated with a denaturation buffer and a renaturation buffer each for 2 minutes and then treated with 1X reaction buffer and 1X enzyme mix.

Referring to FIG. 2, when the target sequence was amplified while thermal cycling was carried out between about 30°C and about 60°C in a method according to an embodiment of the invention, the amplification product was similar to the original template compared to when the control product was used. In FIG. 2, T represents an intact purified human genome, M represents a size marker, and 0.5 h, 1 h, 1.5 h, and 2 h respectively represent 0.5 hours, 1 hour, 1.5 hours, and 2 hours. About 50 ng template DNA, about 100 µM random hexamer primer, about 1 X thermostable Bst DNA polymerase buffer (about 20 mM Tris-HCI, about 10 mM (NH₄)₂SO₄, and about 10 mM KCI, about 2 mM MgSO₄, about 0.1 % Triton X-1 00, pH about 8.8 at about 25°C), and about 10 unit Bst DNA polymerase were mixed, and thermal cycling of incubating at about 30°C for about 20 seconds and incubating at about 60°C for about 40 seconds was repeatedly performed until the time period indicated as above, that is, for about 0.5 hours, about 1 hour, about 1.5 hours, and about 2 hours was lapsed.

As described above, improvement of target sequence amplification efficiency while thermal cycling is carried out may be achieved based on the following mechanism. However, embodiments of the invention are not limited to the mechanism.

First, a random hexamer primer, an exo(-) Bst DNA polymerase, and a human genome were incubated at about 60°C to denature the sequence of a part of the human genome. However, since 60°C is a temperature higher than Tm of the random hexamer primer, the random hexamer primer was not hybridized to the target sequence. When the incubation temperature was decreased to about 30°C, the random hexamer primer was hybridized to the target sequence and elongated. Then, the incubation temperature was increased to about 60°C, the elongated random hexamer primer will be partially denatured and may not separate from the target sequence. When the incubation temperature was decreased from about 60°C to about 30°C, a new random hexamer primer was hybridized to the denatured target sequence part that had been denatured at about 60°C and elongated. As described above, due to thermal cycling, annealing of the primer is increased and thus, the primer elongation is increased. As a result, the amplification efficiency of the target sequence is increased.

According to a method of amplifying a target nucleic acid sequence according to embodiments of the invention, the target nucleic acid sequence may be efficiently amplified.

## Claims

1. A method of amplifying a target nucleic acid sequence, the method comprising:
bringing into contact a primer, a DNA polymerase, and a target nucleic acid sequence in a solution; and
incubating the solution to replicate the target nucleic acid sequence,
wherein the replication of the target nucleic acid sequence results in a replicated strand and during replication, at least one of the replicated strands is displaced from the target sequence by strand displacement replication of another replicated strand,
wherein the incubating is performed while thermal cycling is carried out between an optimal temperature range of the DNA polymerase for its activity and a temperature range in which hybridization between the primer and the target sequence is promoted.

2. The method of claim 1, wherein the primer comprises a random nucleotide sequence or a particular sequence.

3. The method of claim 1 or 2, wherein the primer has a length of about 5 to about 20 bp.

4. The method of claim 3, wherein the primer has a length of about 5 to about 8 bp.

5. The method of claim 4, wherein the primer has a length of about 6 bp.

6. The method of any one of claims 1 to 5, wherein the optimal temperature of the DNA polymerase is higher than a denaturation temperature at which the primer hybridized to the target sequence is denatured, and lower than a denaturation temperature at which a strand replicated by incubation within the temperature range in which hybridization between the primer and the target sequence is promoted, is denatured.

7. The method of any one of claims 1 to 6, wherein the optimal temperature range of the DNA polymerase is in the range of about 30°C to about 75°C , preferably of about 40°C to about 65°C.

8. The method of any one of claims 1 to 7, wherein the hybridization temperature range in which hybridization between the primer and the target sequence is promoted is in the range of about 0°C to about 35°C.

9. The method of any one of claims 1 to 8, wherein the DNA polymerase comprises a ϕ29 DNA polymerase, a Tts DNA polymerase, a M2 DNA polymerase, a VENT^{™} DNA polymerase, a T5 DNA polymerase, a PRD1 DNA polymerase, or a Bst DNA polymerase.

10. The method of any one of claims 1 to 9, wherein the DNA polymerase comprises exo(-) Bst DNA polymerase, the optimal temperature range of the DNA polymerase is about 46°C to about 75°C, and the temperature range at which hybridization between the primer and the target sequence is promoted is in the range of about 4°C to about 35°C.

11. The method of claim 10, wherein the primer comprises a random primer having a length of about 6 bp or a primer having a particular sequence.

12. The method of any one of claims 1 to 11, wherein the primer comprises at least one modified nucleotide whereby said primer is resistant to a nuclease.

13. The method of claim 12, wherein the modified nucleotide comprises a biotinylated nucleotide, a fluorescent nucleotide, 5 methyl dCTP, BrdUTP, or 5-(3-aminoallyl)-2'-deoxyuridine 5'-triphosphate.

## Patentansprüche

1. Verfahren zum Amplifizieren einer Ziel-Nucleinsäuresequenz, wobei das Verfahren umfasst:
In-Kontakt-Bringen eines Primers, einer DNA-Polymerase und einer Ziel-Nucleinsäuresequenz in einer Lösung und
Inkubieren der Lösung, um die Ziel-Nucleinsäuresequenz zu replizieren,
wobei die Replikation der Ziel-Nucleinsäuresequenz zu einem replizierten Strang führt und während der Replikation mindestens einer der replizierten Stränge von der Ziel-Sequenz durch Strangverdrängungsreplikation eines anderen replizierten Strangs verdrängt wird,
wobei die Inkubation durchgeführt wird, während thermisches Cycling zwischen einem optimalen Temperaturbereich der DNA-Polymerase für ihre Aktivität und einem Temperaturbereich, in welchem die Hybridisierung zwischen dem Primer und der Zielsequenz gefördert wird, ausgeführt wird.

2. Verfahren nach Anspruch 1, wobei der Primer eine zufällige Nucleotidsequenz oder eine bestimmte Sequenz umfasst.

3. Verfahren nach Anspruch 1 oder 2, wobei der Primer eine Länge von etwa 5 bis etwa 20 bp aufweist.

4. Verfahren nach Anspruch 3, wobei der Primer eine Länge von etwa 5 bis etwa 8 bp aufweist.

5. Verfahren nach Anspruch 4, wobei der Primer eine Länge von etwa 6 bp aufweist.

6. Verfahren nach einem der Ansprüche 1 - 5, wobei die optimale Temperatur der DNA-Polymerase höher als eine Denaturierungstemperatur ist, bei welcher der an die Zielsequenz hybridisierte Primer denaturiert wird, und niedriger als eine Denaturierungstemperatur, bei welcher ein Strang denaturiert wird, der durch Inkubation in dem Temperaturbereich, in welchem die Hybridisierung zwischen dem Primer und der Zielsequenz gefördert wird, repliziert wurde.

7. Verfahren nach einem der Ansprüche 1 bis 6, wobei der optimale Temperaturbereich der DNA-Polymerase im Bereich von etwa 30°C bis etwa 75°C, vorzugsweise von etwa 40°C bis etwa 65°C, liegt.

8. Verfahren nach einem der Ansprüche 1 bis 7, wobei der Hybridisierungstemperaturbereich, in welchem die Hybridisierung zwischen dem Primer und der Zielsequenz gefördert wird, im Bereich von etwa 0°C bis etwa 35°C liegt.

9. Verfahren nach einem der Ansprüche 1 bis 8, wobei die DNA-Polymerase eine Φ29-DNA-Polymerase, eine Tts-DNA-Polymerase, eine M2-DNA-Polymerase, eine VENT^{™}-DNA-Polymerase, eine T5-DNA-Polymerase, eine PRD1-DNA-Polymerase oder eine Bst-DNA-Polymerase umfasst.

10. Verfahren nach einem der Ansprüche 1 bis 9, wobei die DNA-Polymerase exo(-) Bst DNA-Polymerase umfasst, der optimale Temperaturbereich der DNA-Polymerase etwa 46°C bis etwa 75°C beträgt und der Temperaturbereich, in welchem die Hybridisierung zwischen dem Primer und der Zielsequenz gefördert wird, im Bereich von etwa 4°C bis etwa 35°C liegt.

11. Verfahren nach Anspruch 10, wobei der Primer einen zufälligen Primer mit einer Länge von etwa 6 bp oder einen Primer mit einer bestimmten Sequenz umfasst.

12. Verfahren nach einem der Ansprüche 1 bis 11, wobei der Primer mindestens ein modifiziertes Nucleotid umfasst, wodurch der Primer gegen eine Nuclease resistent ist.

13. Verfahren nach Anspruch 12, wobei das modifizierte Nucleotid ein biotinyliertes Nucleotid, ein fluoreszierendes Nucleotid, 5-Methyl-dCTP, BrdUTP oder 5-(3-Aminoallyl)-2'-desoxyuridin-5'-triphosphat umfasst.

## Revendications

1. Procédé d'amplification d'une séquence d'acide nucléique cible, le procédé comprenant :
la mise en contact d'une amorce, d'une ADN polymérase, et d'une séquence d'acide nucléique cible dans une solution ; et
l'incubation de la solution pour répliquer la séquence d'acide nucléique cible,
dans lequel la réplication de la séquence d'acide nucléique cible donne un brin répliqué et pendant la réplication, au moins un des brins répliqués est déplacé de la séquence cible par la réplication, par déplacement de brin, d'un autre brin répliqué,
dans lequel l'incubation est effectuée pendant que des cycles thermiques sont exécutés entre une plage de température optimale de l'ADN polymérase pour son activité et une plage de température dans laquelle l'hybridation entre l'amorce et la séquence cible est favorisée.

2. Procédé selon la revendication 1, dans lequel l'amorce comprend une séquence nucléotidique aléatoire ou une séquence particulière.

3. Procédé selon la revendication 1 ou 2, dans lequel l'amorce a une longueur d'environ 5 à environ 20 pb.

4. Procédé selon la revendication 3, dans lequel l'amorce a une longueur d'environ 5 à environ 8 pb.

5. Procédé selon la revendication 4, dans lequel l'amorce a une longueur d'environ 6 pb.

6. Procédé selon l'une quelconque des revendications 1 à 5, dans lequel la température optimale de l'ADN polymérase est plus élevée qu'une température de dénaturation à laquelle l'amorce hybridée à la séquence cible est dénaturée, et plus basse qu'une température de dénaturation à laquelle un brin répliqué par incubation dans la plage de température dans laquelle l'hybridation entre l'amorce et la séquence cible est favorisée, est dénaturé.

7. Procédé selon l'une quelconque des revendications 1 à 6, dans lequel la plage de température optimale de l'ADN polymérase va d'environ 30 °C à environ 75 °C, de préférence d'environ 40 °C à environ 65 °C.

8. Procédé selon l'une quelconque des revendications 1 à 7, dans lequel la plage de température d'hybridation dans laquelle l'hybridation entre l'amorce et la séquence cible est favorisée va d'environ 0 °C à environ 35 °C.

9. Procédé selon l'une quelconque des revendications 1 à 8, dans lequel l'ADN polymérase comprend une ADN polymérase de ϕ29, une ADN polymérase de Tts, une ADN polymérase de M2, une ADN polymérase VENT^{™}, une ADN polymérase de T5, une ADN polymérase de PRD1, ou une ADN polymérase de Bst.

10. Procédé selon l'une quelconque des revendications 1 à 9, dans lequel l'ADN polymérase comprend une ADN polymérase de Bst exo(-), la plage de température optimale de l'ADN polymérase va d'environ 46 °C à environ 75 °C, et la plage de température à laquelle l'hybridation entre l'amorce et la séquence cible est favorisée va d'environ 4 °C à environ 35 °C.

11. Procédé selon la revendication 10, dans lequel l'amorce comprend une amorce aléatoire ayant une longueur d'environ 6 pb ou une amorce ayant une séquence particulière.

12. Procédé selon l'une quelconque des revendications 1 à 11, dans lequel l'amorce comprend au moins un nucléotide modifié, grâce à quoi ladite amorce est résistante à une nucléase.

13. Procédé selon la revendication 12, dans lequel le nucléotide modifié comprend un nucléotide biotinylé, un nucléotide fluorescent, du 5-méthyl-dCTP, du Br-dUTP, ou du 5-(3-aminoallyl)-2'-désoxyuridine-5'-triphosphate.
